# EUROPEAN PATENT APPLICATION

(11) **EP 2 364 696 A1**
(43) Date of publication of application: **14.09.2011**
(21) Application number: 11169021.0
(22) Date of filing: 31.10.2007
(51) Int. Cl.: A61K 9/72, A61K 38/28, A61K 31/137, A61K 31/573, A61K 47/08, A61K 9/16, A61K 9/14, A61K 9/19

(54) **Formulations for delivery via pressurised metered dose inhalers**

(30) Priority: 31.10.2006 GB 0621707
(62) Divisional of application: 07824878.8
(71) Applicant: SCHOOL OF PHARMACY, UNIVERSITY OF LONDON, London WC1N 1AX (GB)
(72) Inventor: Kellaway, Ian Walter, Salterton, Devon EX9 7AP (GB); Taylor, Kevin, London, WC1N 1AX (GB); Nyambura, Bildad Kimani, London, SE28 8DZ (GB)
(74) Representative: Jones, Helen M.M.

(57) **Abstract**

A formulation suitable for delivery from a pressurised metered dose inhaler comprises a hydrofluoroalkane liquid, which is pharmaceutically acceptable, for instance HFA 134a or HFA 227, a drug to be delivered to the lung which is insoluble in the hydrofluoroalkane, the drug being in particulate form in the composition, further containing a suspension stabiliser which is a non-toxic essential oil miscible with the hydrofluoroalkane liquid at room temperature. The essential oil is preferably one having some amphiphilic property, such as a ketone or aldehyde compound. Insulin may be formulated for inhalation.

## Description

The present invention relates to compositions which are suitable for administering to humans or animals via pressurised metered dose inhalers. The compositions include a propellant in which an active is suspended, which further includes a suspension stabiliser.

Pressurised metered dose inhalers (pMDIs) allow delivery of a predetermined dose of a composition from the aerosol-like device. Inside the pressurised container is a propellant, under pressure in the form of a liquid. The propellant suspends the active agent during delivery from the device, and evaporates to an extent prior to the active reaching the throat. The active is generally insoluble in the propellant liquid and as such is in the form of suspended particles in the liquid.

In order for a dose expelled from the pMDI to contain the appropriate dose of active ingredient, it is crucial for the active to be stably suspended in the liquid. It has been suggested that the particles may be coated with surfactant so as to stabilise the suspension. However many of the traditional and pharmaceutically acceptable surfactants in use today are insoluble in the liquids used as propellants in pMDIs. It has been necessary to use a co-solvent which is miscible with the propellant and dissolves the surfactant. It would be desirable to avoid the need for co-solvents and avoid the presence of such surfactants.

WO-A-9640089 discloses a pharmaceutical composition for aerosol delivery comprising a medicament, a halogenated alkane propellant and a biocompatible C₁₆₊⁻ unsaturated vegetable oil and a method for preparing such compositions. Unwanted aggregation of the medicament is prevented without the use of surfactants, protective colloids or cosolvents by the incorporation of the vegetable oil.

EP-A-1166774 discloses the use of a flavouring oil in a pharmaceutical aerosol formulation. The flavouring oil, as well as masking unpleasant tastes of sublingual compositions, acts as a lubricant of the valve of the dispersing device used to administer said formulation. Preferably the flavouring oil is a volatile oil and also acts as a penetration enhancer. An example is peppermint oil which is used in conjunction with ethanol, the carrier of an unspecified active.

EP-A-0372777 discloses a pharmaceutical composition for aerosol delivery e.g. for inhalation, comprising a medicament, 1,1,1,2-tetrafluoroethane (HFA 134a), a surface active agent and at least one compound having higher polarity than 1,1,1,2-tetrafluoroethane such as an alcohol, a hydrocarbon or another propellant. The examples use ethanol and n-pentane as the additive and all the worked examples include also surfactant.

EP-A-1219293 discloses a pharmaceutical composition for aerosol delivery comprising a medicament, a hydrofluoroalkane, a cosolvent and a low volatility component. The low volatility component increases the mean median, aerodynamic diameter of the aerosol particles on activation of the inhaler. The cosolvent is generally ethanol or a glycol. The low volatility component may be a vegetable oil, a fatty acid, a polyethylene glycol or glycerol.

US5502076 discloses the use of vitamin E acetate, C₃-linked triesters, glycerin, t-butanol, and transesterified oil/polyethylene glycols as effective dispersing agents for use with hydrofluoroalkanes, for use in metered-dose inhalers.

US-A-6123924 discloses a pressurised aerosol inhalation composition comprising a liquefied hydrofluoroalkane, a powdered medicament dispersible to form a suspension in the liquefied hydrofluoroalkane and polyvinylpyrrolidone as stabiliser. The compositions additionally comprise polyethoxylated valve lubricants and flavouring excipients such as peppermint oil and menthol and generally also ethanol and/or propanol which increase the solubility of the polymer.

WO-A-9111173 discloses a pressurised aerosol composition comprising a liquefied hydrofluorocarbon propellant containing substantially no non-hydrofluorocarbon solvent, having dispersed therein a medicament and a fluorinated surfactant.

WO-A-9104011 discloses a self-propelling, powder dispensing aerosol comprising medicament coated with a non-perfluorinated surface-active dispersing agent suspended in an aerosol propellant in which the non-perfluorinated surface-active dispersing agent is substantially insoluble. The dispersing agent may be a vegetable oil such as corn, olive, cotton seed or sunflower seed oil.

Pharmaceutically acceptable propellants used in pMDIs are hydrofluoroalkane 227 and 134a.

Many essential oils are useful as pharmaceutically acceptable excipients for a range of pharmaceutical compositions. Essential oils themselves may have useful therapeutic properties. Essential oils may well have utility in compositions which are inhaled, either through the nose or through the mouth. However, to the inventors' knowledge the use of essential oils as a suspension stabiliser for a drug in a pressurised metered dose inhaler has not been described.

There is provided according to one aspect of the invention a new metered dose inhaler containing a composition comprising a pharmaceutically acceptable hydrofluoroalkane liquid propellant, a drug to be delivered to the lung which is insoluble in the propellant and is in particulate form suspended in the propellant and an effective drug suspending amount of a pharmaceutically acceptable essential oil which is miscible with the propellant.

In another aspect of the invention there is provided the new use of a pharmaceutically acceptable essential oil to stabilise in a pMDI a suspension of a particulate drug in a pharmaceutically acceptable hydrofluoroalkane liquid in which the drug is insoluble and with which the essential oil is miscible.

In another aspect of the invention there is provided new use of a combination of a pharmaceutically acceptable hydrofluoroalkane liquid, a drug and a pharmaceutically acceptable essential oil in the manufacture of a composition for administration to the lung of an animal subject via inhalation, wherein the drug is in particulate form in the composition, the particles of drug are suspended in the hydrofluoroalkane liquid, the essential oil is miscible with the hydrofluoroalkane and the suspension is stabilised by the essential oil.

The hydrofluoroalkane liquid in the composition is preferably selected from hydrofluoroalkane 227 and134a.

The non-toxic essential oil used in the composition of the invention may generally be defined as a liquid which is substantially immiscible with water at room temperature, and is a liquid at room temperature. Essential oils are volatile oils comprised mainly of mono- and sesquiterpene hydrocarbons and their oxygen derivatives. They are products of distillation, expression or solvent extraction of plants, including flowers, leaves, wood and grasses, or they may be produced synthetically. They comply with the European monograph for Essential Oils. The oil may be a single compound, or may be a mixture of compounds. The term "essential oils" is intended to include the following examples, though the invention is not limited to these examples: allspice berry, amber essence, anise seed, arnica, balsam of peru, basil, bay, bay leaf, bergamot, bois de rose (rosewood), cajeput, calendula (marigold pot), white camphor, caraway seed, cardamon, carrot seed, cedarwood, celery, chamomile, chamomile, cinnamon, citronella, clary sage, clovebud, coriander, cumin, cypress, eucalyptus, fennel, siberian fir needle, frankincense(olibanum oil), garlic, rose geranium, ginger, grapefruit, hyssop, jasmine, jojoba, juniper berry, lavender, lemon, lemongrass, lime, marjoram, mentol, mugwort, mullein flower, myrrh gum, bigarade neroli, nutmeg, bitter orange, sweet orange, oregano palmarosa, patchouly, pennyroyal, black pepper, peppermint, petite grain, pine needle, poke root, rose absolute, rosehip seed, rosemary, sage, dalmation sage, sandalwood, sassafras, spearmint, spikenard, tangerine, tea tree, thuja (cedarleaf), thyme, vanilla extract, vetivert, wintergreen, witch hazel (hamamelia) extract, and ylang ylang (cananga) extract and isolated or synthesised components of these, such as 2,6-dimethyl-2,4,6-octatriene; 4-propenylanisole; benzyl-3-phenylpropenoic acid; 1,7,7-trimethylbicyclo[2.2.1]heptan-2-ol; 2,2-dimethyl-3-methylenebicyclo[2.2.1]heptane; 1,7,7-trimethylbicyclo[2.2.1]heptane; trans-8-methyl-n-vanillyl-6-nonenamide; 2,2,5-trimethylbicyclo[4.1.0]hept-5-ene; 5-isopropyl-2-methylphenol; p-mentha-6,8-dien-2-ol; p-mentha-6,8-dien-2-one;. beta-caryophyllene; 3-phenylpropenaldehyde; mixed geranial and neral; 3,7-dimethyl-6-octenal; 3,7-dimethyl-6-octen-1-ol; 4-allylanisole; ethyl-3 phenylpropenoic acid; 3-ethoxy-4-hydroxybenzaldehyde; 1,8-cineole; 4-allyl-2 methoxyphenol; 3,7,11-trimethyl-2,6,10-dodecatrien-1-ol; 1,3,3-trimethylbicyclo[2.2.1]heptan-2-ol; 1, 3,3-trimethylbicyclo[2.2.1]heptan-2-one; trans-3,7-dimethyl-2,6-octadien-1-ol; trans-3,7-dimethyl-2,6-octadien-1-yl acetate; 3-methyl-2-(2 pentenyl)-2-cyclopenten-1-one; p-mentha-1,8-diene; 3,7-dimethyl-1,6-octadien-3-ol; 3,7-dimethyl-1,6-octadien-3-yl acetate; p-menthan-3-ol; p-menthan-3-one; methyl-2-aminobenzoate; methyl-3-oxo-2-(2-pentenyl)-cyclopentane acetate; methyl-2-hydroxybenzoate; 7-methyl-3-methylene-1,6-octadiene; cis-3,7-dimethyl-2,6-octadien-1ol; 2,6,6-trimethylbicyclo[3.1.1]hept-2-ene; 6,6-dimethyl-2-methylenebicyclo[3.1.1]heptane; p-menth-4-(8)-en-3-one; p-menth-1-en-4-ol; p-mentha-1,3-diene; p-menth-1-en-8-ol; 2-isopropyl-5-methylphenol; citral; cinnamaldehyde and cineole.

Of these essential oils, those which are labeled as GRAS (Generally Regarded As Safe) for certain modes of application are particularly preferred.

Although the oil used in the invention must be substantially immiscible with water, it is generally found to be useful for the oil to have some amphiphilic properties. The oil may comprise a relatively hydrophobic portion and a relatively hydrophilic portion. The hydrophobic portion generally comprises one or more C₆-₂₄-alkyl groups, preferably a single alkyl group, more preferably a C₆-₁₆ alkyl group. A relatively hydrophilic portion may, for instance, comprise a ketonic or aldehydic carbonyl group. Preferably, the oil is amphiphilic and the hydrophobic portion comprises at least one C₆-₂₄ alkyl chain and the hydrophilic portion preferably comprises a ketonic or aldehyde carbonyl group. Oils which have some amphiphilic character are believed to tend to localise at the surface of the particles of pharmaceutically active compound and act to stabilise their suspension in the hydrofluoroalkane.

In a preferred embodiment of the invention the oil is selected from citral, menthol, eucalyptus oil, cinnamaldehyde and cineole. Mixtures may be used.

The essential oils acts as a suspending agent. Its effect is to inhibit agglomeration of the drug particles in the suspension in liquefied HFA. The effect may be observed for instance by determining the particle size of suspended particles formed in the absence and in the presence of the essential oil. It may be possible to avoid the presence of all other additives which could affect the suspension, such as liquids like alcohols, glycols and esters, or surfactants or copropellants.

Preferably the lever of other liquids is less than 20% by volume of the composition preferably less than 5% or less than 1% by volume.

In the preferred composition of the invention the volume ratio of essential oil to hydrofluoroalkane is in the range 1 to 10,000 to 10,000 to 1, preferably in the range 1 to 100 to 1 to 2. The amount of essential oil may depend on the particle size and solids concentration of the active particles as well as the nature of the oil, the HFA and the active. It may be determined by the person skilled in the art.

The invention has two areas of utility, for two different classes of pharmaceutical active. The invention is of utility for formulating active compounds which are water soluble, that is have a water solubility of at least 0.1 mg/ml at room temperature. For example, it is of particular benefit for compounds which are sensitive to their environment and are formulated with solid protectants, e.g. lyoprotectants, such as a sugar. Sugars used as a lyoprotectant are generally selected from lactose and trehalose, but other sugars may also be used. The particles which are suspended in the liquid comprise both the protectant and the active. The particles themselves may be formed using the process of freeze-drying water-in-oil emulsions containing the pharmaceutical active and protectant in aqueous solution in the dispersed phase followed by washing. Alternatively, more conventional processing technologies for producing particles with desired characteristics (e.g. milling, precipitation, crystallization, polymerization, spray-drying, supercritical fluid processing, solvent diffusion/evaporation, etc) may be employed.

In this aspect of the invention a water-soluble active is, for instance, a peptide or a protein, for instance an enzyme or a hormone, or a nucleic acid, for instance siRNA and DNA, such as gene therapy vectors and gene vaccines. The invention has been found to be of particular utility for formulating insulin. It is of particular utility for formulating proteins where the active particles comprise a protectant, preferably lactose.

In this aspect of the invention the particles suspended in the composition have mean particle size in the range 0.01 to 20 µm preferably in the range 0.2 to 10 µm. Particles which are too large are deposited in the throat rather than reaching the lung. Particles which are too small may be exhaled.

The other type of active for which the invention has particular utility is for formulating relatively water-insoluble compounds. Such compounds may be defined as having water solubility less then 0.1 mg/ml at room temperature. Such compounds should preferably have solubility in the stabiliser of at least 0.5 mg/ml at room temperature. Generally such compounds have solubility in the hydrofluoroalkane less than 0.1 mg/ml. Where the active has a higher solubility than this maximum in the hydrofluoroalkane, there should generally be no need for additional stabiliser and so the invention has little utility.

The relatively water-insoluble actives are often selected from steroids, corticosteroids, bronchodilators, beta-2 agonists, antibiotics, anti-microbials, antivirals, muscarinic antagonists, phosphodiesterase inhibitorsand antihistamines. The invention is of utility for formulating an active selected from, for instance, salmeterol, terbutaline, cromolyns, beclometasone, budesonide, mometasone, ciclesonide, triamcinolone, fluticasone, rofleponide, salbutamol, formeterol, oxitropium, roflumilast and pharmaceutically acceptable salts and esters of any of these.

The composition should have a concentration of active selected according to the therapeutic activity thereof and the volume dosage of composition usefully administered by the pMDI. The drug concentration is preferable in the range 0.001 to 10% w/w based on the total composition. A suitable unit dose administered via a pMDI contains 30 to 100 µl liquid composition. A suitable pharmaceutical active content for such a dose is in the range 0.001 to 10 mg/100 µl, depending on the activity of the pharmaceutical agent, the age and weight of the patient etc. The inhaler is preferably adapted to deliver a dose of composition containing drug in an amount in the range of 0.001 to 10 mg drug per actuation. The person skilled in the art is generally able to select suitable concentrations for the invention. The invention allows stable compositions to be created having concentrations of up to 10 mg/100 µl active, allowing carefully controlled dosages to be delivered. For instance, a dose of composition administered per actuation of a pMDI contains in the range of 30 to 600 µg of pharmaceutical active per actuation.

The essential oil may be used as the sole suspending agent in the composition. Its stabilising properties may allow other conventional components such as fixed oils (vegetable oils), alcohols, glycols, surfactants and polymers to be omitted from the compositions. The essential oil often prevents agglomeration of the drug particles to a sufficient degree to be used alone. Preferably the composition is substantially free of fixed oils, although small quantities may present for instance as diluent for essential oil or as residue from an active as supplied. Preferably the level of fixed oils is less than 5% by volume, more preferably less than 1% by volume.

Where polymer such as PVP is used as an additional suspending agent it is generally present in an amount in the range 0.01 to 5% w/v, preferably 0.1 to 2% w/v.

It is preferred to avoid administering alcohols and glycols and so the compositions are preferably substantially free of lower alcohols and glycols (C₂₋₆), for instance containing less than 10% by volume, preferably less than 5% by volume, for instance less than 1% by volume.

It is also possible, by the use of essential oils in the invention, to avoid the inclusion of surfactants. However for some actives, the use of pharmaceutically acceptable surfactants may be provide additional control over the particle size or drug. For instance there may be surfactant used in the composition in an amount of 0.01 to 5% w/v, preferably less than 1% w/v. Most preferably the composition is substantially free of surfactant, that is there is less than 0.01% w/v surfactant present.

There is also provided in the invention a method of producing the filled inhaler in which pharmaceutical active is dispersed into the suspension stabiliser to form a stabiliser dispersion, and the stabiliser dispersion is mixed with hydrofluoroalkane, usually in the device and under pressure.

Where the method is used for a pharmaceutical active which is soluble in the suspension stabiliser, the active is dissolved into the suspension stabiliser to form a solution and the solution is added to the hydrofluoroalkane. Upon addition to the non-solvent for the therapeutic active (i.e. the hydrofluoroalkane), drug precipitate is formed. The presence of the suspension stabiliser maintains the particles of the precipitate in suspension.

Where the pharmaceutical active is insoluble in the suspension stabiliser, particles of the desired size for the final product are dispersed into the liquid suspension stabiliser, to form a stable suspension, and the suspension is then added to hydrofluoroalkane to form the stable final composition. In this embodiment the suspended particles may contain protectant which are generally sugars such as lactose and trehalose, and other appropriate excipients as well as active.

The present invention also encompasses novel compositions a) a pressurised composition for inhalation comprising a pharmaceutically acceptable hydrofluoroalkane liquid propellant, insulin in suspended particulate form and cineole and b) a pressurised composition inhalation for comprising a pharmaceutically acceptable hydrofluoroalkane liquid propellant, a steroid or corticosteroid for delivery to the lung and citral.

The invention is illustrated in the worked examples. The results of some of the examples are illustrated in the figures as follows:
Figure 1 shows the particle size determination results on products of Example 1;
Figure 2 shows the effect on the aerolisation results by increasing oil concentration for products of the invention as described in Example 1;
Figure 3 shows the variation of aerolisation results upon changing the concentration of active in the process as described in Example 1;
Figures 4 and 5 show the results of Example 2.

### Example 1

### INSULIN-BASED COMPOSITIONS WITH CINEOLE STABILISER

Insulin nanoparticles were prepared using an emulsion template method. 80 mg insulin and 20 mg lactose were dissolved into 1 ml of 0.1 M HCl. 2 g lecithin (phosphatidylcholine surfactant) was weighed separately and dissolved in 7 ml chloroform to form the oily phase. The aqueous phase was added dropwise into the oil phase while homogenizing at low speed (10,000 rpm) followed by high speed homogenization (24,000 rpm) for 5 min. The emulsion formed was immediately snap-frozen using liquid nitrogen to immobilise the disperse phase. The frozen emulsions were transferred into a vacuum proof glass jar (Girovac Ltd., North Walsham, Norfolk, UK) and attached to the freeze-drier (Drywinner 110, Heto-Holten A/S, Gydevang, Denmark) set at -110°C. Freeze-drying was performed for a minimum of 12 h to remove water from the frozen microscopic aqueous droplets.

Following the freeze-drying stage, dry matter containing nanoparticles covered with surfactant (lecithin) was obtained. This was washed to remove lecithin by the following steps. The dry matter was suspended in 0.5% v/v triethylamine (TEA) in dichloromethane in which insulin and lactose were insoluble while lecithin was freely soluble, therefore preserving the structure of the nanoparticles. These nanoparticles were separated from free surfactant by centrifugation (3K30 Refrigerated centrifuge, Sigma Laborzentrifuges GmbH, Osterode am Harz, Germany). The sedimentation conditions were 17,000 rpm at 25°C. 50 ml Oakridge Teflon (Trade Mark) centrifuge tubes (Nalge-Nunc Inc., Rochester, NY, USA) were selected for centrifugation owing to their excellent solvent compatibility and ease of nanoparticle collection from the non-stick surface. The solvent plus surfactants were decanted and the sediments which comprised nanoparticles were collected. The washing process was repeated twice.

The sediment of nanoparticles was suspended in 5 ml of dichloromethane and 0.25 ml cineole was added. The suspension formed was vortexed to ensure homogeneity. Dichloromethane was removed by evaporation under vacuum using a Rotavapor® (Büchi, Switzerland) set at 35°C for 5 min. A paste of nanoparticles moistened by cineole was obtained which was subsequently cold filled, oil placed in vials and cooled to a temperature below the boiling point of HFA 134a, then chilled (condensed) HFA 134a was added and the vial closed. The solid concentration (i.e. insulin plus lactose) of the filled vials was 1 % w/w.

Application of an anti-foaming agent to the emulsion (glyceryl monoleate) was investigated with an objective of reducing the size of the nanoparticles. This was achieved by dissolving 1 g of glyceryl monoleate and 2 g of lecithin in 7 ml of chloroform to form the oily phase. However, all other processing conditions were maintained as described above.

The formulation was subsequently assessed within two days of manufacture to determine the particle size using photon correlation spectroscopy (PCS), and the morphology of the particles was examined using scanning electron microscopy (SEM).

The formulations were also assessed for the integrity of insulin using gel permeation chromatography (GPC), high performance liquid chromatography (HPLC), circular dichroism and fluorescence spectroscopy.

Furthermore, aerolisation characterisation of the nanoparticles was carried out using a multistage liquid impinger (MSLI) set at 60L/min (effective cut-off diameters: stage 1 = 13 µm, stage 2 = 6.8 µm, stage 3 = 3.1 µm and stage 4 = 1.7 µm). The MSLI separates aerosol particles in a moving airstream on the basis of their aerodynamic diameters and allows an estimate of the fine particle fraction (FPF) of the aerosol emitted from a pMDI. In this work, FPF refers to the fraction of the inhaler output that has an aerodynamic diameter less than about 1.7 µm. This represents the fraction of the drug dose that can reach the alveolar region of the lung where systemic drug absorption occurs.

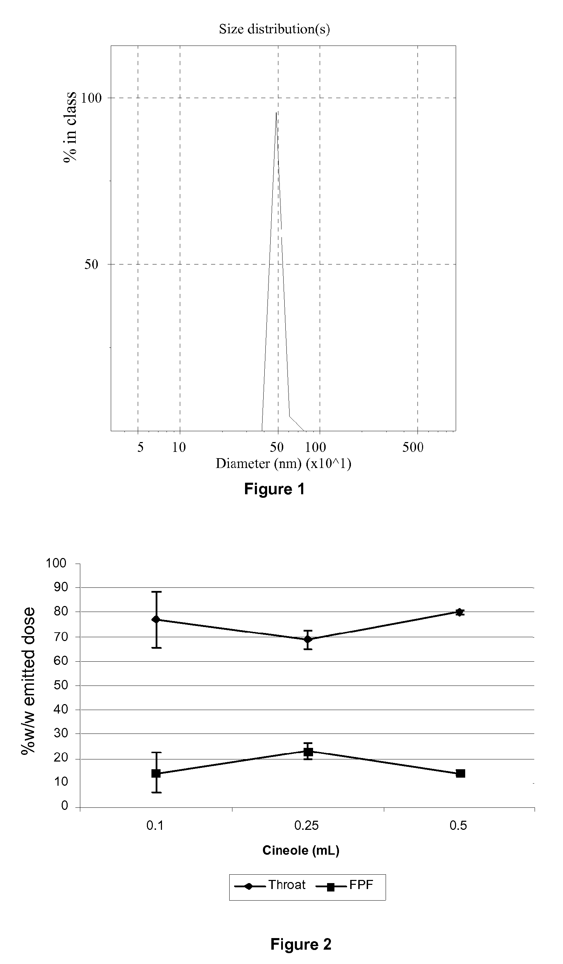

The PCS results for the product produced without glyceryl monooleate (Figure 1) indicate that the nanoparticles have a z-average diameter of around 550 nm with a narrow size distribution (polydispersity index 0.084 as determined by the PCS device). The SEM micrographs showed that the nanoparticles were substantially spherical in shape. The GPC and HPLC chromatograms, compared to standard insulin, show that the products are very similar to standard insulin, suggesting that insulin is not chemically degraded during the formulation process.

Far-UV circular dichroism spectra indicate that the secondary structures of insulin are retained after processing. There is no significant difference (p<0.05) between spectra, and the secondary structure composition of unprocessed (control material) and processed (from the nanoparticles after delivery from the pMDI) were comparable.

Control insulin, insulin from the nanoparticles after freeze-drying and insulin recovered following actuation of the pMDI formulation produced similar near-UV CD spectra, with a peak at around 275 nm. This indicates retention of tertiary structures of insulin after processing. This is confirmed from fluorescence spectra of insulin samples after delivery from the pMDI, and before and after denaturation with 6M guanidine hydrochloride, as compared with standard insulin under the same conditions. The tests on the aerolisation characteristics of the compositions show that there was an optimal stabiliser level. At concentrations of stabiliser which are too high, there may be an effect of reduction of the HFA evaporation from the spray plume, which leads to larger droplets with a high mass and consequently higher levels of deposition in the throat. With very low stabiliser concentrations, the nanoparticles are inadequately dispersed, possibly leading to aggregation and, again, higher levels of throat deposition. With a level of 0.25 ml stabiliser per batch the FPF improves. The results of three different levels of stabiliser are shown in Figure 2 which shows the effect of cineole concentration (ml/batch; a batch is equivalent to 100mg nanoparticle (insulin plus lactose) formulation in 10g HFA 134a) on FPF and throat deposition of nanoparticles.

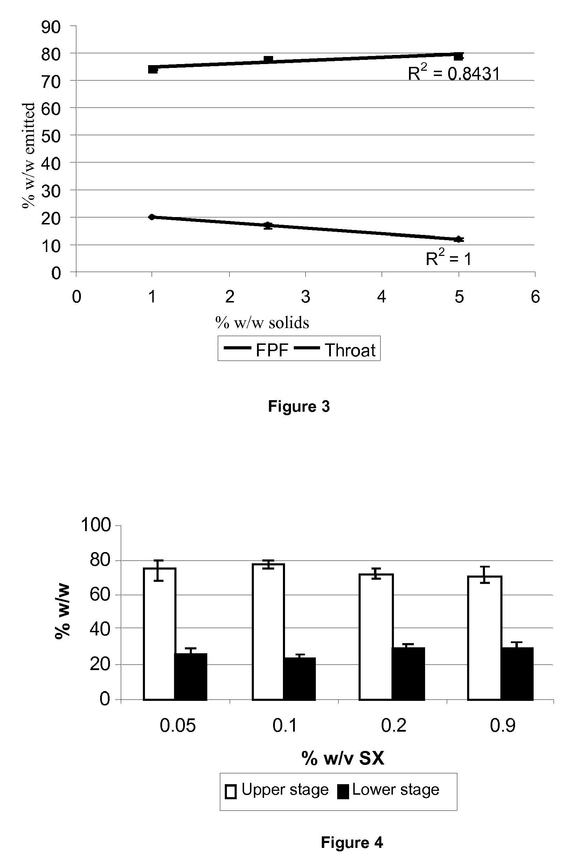

Figure 3 shows the FPF and throat deposition of pMDI formulations containing 1%, 2.5% and 5% concentration of insulin nanoparticles in HFA 134a each with cineole at a level of 0.25 ml/batch of 10 g HFA).

It was also found that throat deposition decreased as the concentration of solid decreased, while FPF increased with decreasing solid concentration (Figure 3).

The size of the nanoparticles was reduced to z-average diameter of around 347 nm when glyceryl monoleate was added to the oil phase during the production of 1% formulations of nanoparticles. These nanoparticles were used to formulate the optimized pMDI formulation.

Further studies were performed investigating the aerolisation of the optimized formulation. The emitted dose per actuation was approximately 0.55 mg total solids that yield 0.44 +/- 0.04 mg/actuation insulin, based on the insulin content in the nanoparticles being 80%. The formulation had around 45% insulin weight of emitted dose (ex-actuator) delivered as FPF.

### Example 2

### SALMETEROL XINAFOATE (SX)-BASED COMPOSITIONS WITH CITRAL SUSPENDING AGENT

SX was weighed and dissolved in a known amount of chilled citral. SX has an aqueous solubility of 66-81 µg/ml (Tong, HY, et al, Pharm. Res. (2001) 18, 852-858), i.e. it is not "water-soluble" in the terms of the present specification. The solution (see below for amount/concentration) was added dropwise in 10 ml of chilled HFA 134a while homogenizing at a low speed (10,000 rpm). A 63 µl actuation metering valve (Valois DF60 MK42; Valois, France) was immediately crimped onto the pMDI canister (using manual bottle crimper 3000, Aero-Tech Laboratory Equipment Company, USA). The amount of citral used was varied from 2 to 33% v/v while the SX concentration in the formulation was varied from 0.05 to 0.9% w/v based on the entire formulation. The pMDI vials were sonicated (XB6 Grant Instruments Ltd., UK) for approximately 1 min and the stability of the suspension was assessed visually. Formulations containing 0.05, 0.1, 0.2, & 0.9% w/v SX and 2, 9 & 23% v/v citral were assessed for their aerosolisation characteristics using a two-stage (twin) impinger set at 60L/min (effective cut-off diameter between stages = 6.4 µm). The amount of SX in the upper and lower stage of the twin impinger was assessed using a high performance liquid chromatography (HPLC) set with the conditions outlined in Table 1.

**Table 1: HPLC settings**

| **Component** | **Setting** |
|---|---|
| Injection volume | 20 µl |
| Flow rate | 1 ml/min |
| Retention time | ~ 2.2 min |
| Temperature | 40°C |
| UV detector | 228 nm |
| Column | Kromasil C18, ODS - 2 (5 im, 250 mm x 4.6 mm id) |
| Mobile Phase | Methanol:Acetonitrile:De-ionised water (30:30:40 v/v) containing 0.6% w/v ammonium acetate and 0.2% w/v tetrabutylammonium |

SX particles suspended in HFA134a were formed instantaneously when SX in citral solution was introduced to HFA 134a in all concentrations investigated as judged by observing the suspension in the sealed glass vials.

Aerosolisation studies were carried out to show the effect of changing the drug concentration on drug deposited in the upper and lower stages with citral in an amount of 9% v/v. The results showed that the % w/w of drug deposited in the lower and upper stages of twin impinger was not significantly different (p < 0.05) when formulations with different SX concentration were assessed (Figure 4). However, the amount of emitted dose increased with the increasing drug concentration in the formulation.

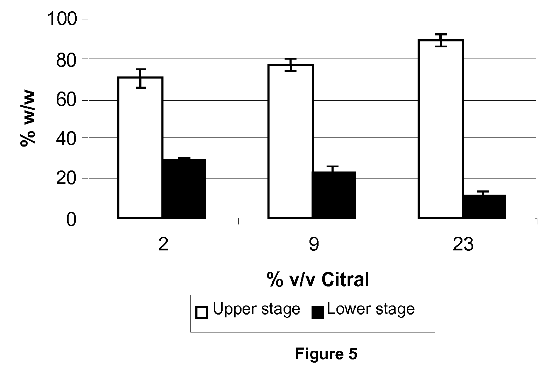

The effect of changing the citral concentration on the aerolisation of the formulations is shown in Figure 5 (using the 0.1 % SX composition). An increase in citral concentration in the formulation led to a decreased amount of SX (%w/w) deposited at the lower (FPF)stage of the twin impinger, whilst the amount of drug deposited in the upper stage of the impinger increased with increased citral in the formulation (Figure 5). The effective cut-off diameter between stages for the twin impinger is 6.4 µm, thus FPFs₆₄ µm in up to of 30% were demonstrated using this dispersion technique (Figures 4 and 5) with 2% v/v citral.

## Claims

1. A pressurised metered dose inhaler comprising a composition comprising a pharmaceutically acceptable hydrofluoroalkane liquid propellant, a drug to be delivered to the lung which is insoluble in the hydrofluoroalkane liquid, in particulate form, and a suspension stabiliser which is a pharmaceutically acceptable essential oil which is miscible with the hydrofluoroalkane liquid at room temperature, wherein the volume ratio of stabiliser to hydrofluoroalkane is in the range 1:100 to 1:2.

2. An inhaler according to claim 1, in which the particles have an average diameter in the range 0.01 to 20 µm.

3. An inhaler according to claim 1 or 2, in which the drug is a compound which is water-soluble, having water-solubility more than 0.1 mg/ml.

4. An inhaler according to any preceding claim, in which the particles containing drug also comprise a solid protectant, preferably a lyoprotectant, more preferably a sugar, most preferably selected from lactose and trehalose.

5. An inhaler according to claim 1 or 2, in which the drug is a compound having a water-solubility less than 0.1 mg/ml (room temperature), a solubility in the stabiliser of at least 0.5 mg/ml (room temperature), and a solubility in the hydrofluoroalkane less than 0.1 mg/ml (room temperature).

6. An inhaler according to claim 5, in which the drug is selected from steroids, corticosteroids, bronchodilators, beta-2 agonists, antibiotics, antimicrobials, antivirals and antihistamines.

7. An inhaler according to claim 1 or 2, in which the drug is selected from salmeterol, terbutaline, cromolyns, beclometasone, budesonide, mometasone, ciclesonide, triamcinolone, fluticasone, rofleponide, salbutamol, formeterol, oxitropium, roflumilast and pharmaceutically acceptable salts and esters of any of these.

8. An inhaler according to any preceding claim, in which the oil is selected from citral, menthol, eucalyptus oil, cinnamaldehyde and cineole and/or in which the hydrofluoroalkane is HFA 134a or HFA 227.

9. A method of producing an inhaler according to any of claims 1 to 8, in which drug is dispersed into the suspension stabiliser to form a dispersion, and the dispersion is added to hydrofluoroalkane to form a suspension, the suspension is put in a container and the container is closed in such a manner that the contents are pressurised at room temperature.

10. A method according to claim 9, in which the drug is soluble in the suspension stabiliser to an extent of no more than 0.1 mg/ml and is dispersed in the form of particles into the suspension stabiliser to form a suspension of the particles in the suspension stabiliser, and the suspension is combined with the hydrofluoroalkane.

11. A method according to claims 9 or 10, which comprises preliminary steps: forming an aqueous solution of drug and protectant; forming a water-in-oil emulsion of aqueous solution of drug and protectant into a continuous oil phase; and drying the emulsion to form particles, preferably in which the emulsion is dried by a spray-drying or lyophilisation process.

12. A method according to claim 9, in which the drug is soluble in the suspension stabiliser at a level of at least 0.5 mg/ml, wherein the drug is dissolved into the suspension stabiliser to form a solution and the solution is combined with the hydrofluoroalkane, whereby a drug precipitate is formed.

13. A method according to claim 12, in which the dispersion and the hydrofluoroalkane are mixed together under pressure in a container which is subsequently closed to form a pressurised metered dose inhaler.

14. A use of a pharmaceutically acceptable essential oil to stabilise in a pMDI a suspension of a particulate drug in a pharmaceutically acceptable hydrofluoroalkane liquid in which the drug is insoluble and with which the essential oil is miscible.

15. A use of a combination of a pharmaceutically acceptable hydrofluoroalkane liquid, a drug and a pharmaceutically acceptable essential oil in the manufacture of a composition for administration to the lung of an animal subject via inhalation, wherein the drug is in particulate form in the composition, the particles of drug are suspended in the hydrofluoroalkane liquid, the essential oil is miscible with the hydrofluoroalkane and the suspension is stabilised by the essential oil.
